# EUROPEAN PATENT APPLICATION

(11) **EP 4 775 126 A1**
(43) Date of publication of application: **15.07.2026**
(21) Application number: 25151673.8
(22) Date of filing: 14.01.2025
(51) Int. Cl.: A61B 1/015, A61B 18/00

(54) **CONTROL SYSTEM AND METHOD FOR CONTROLLING AN OPERATION PERFORMED VIA A MEDICAL INSTRUMENT**

(71) Applicant: OLYMPUS SURGICAL TECHNOLOGIES EUROPE Olympus Winter & Ibe GmbH, 22045 Hamburg (DE)
(72) Inventor: WOLTER, Michael, 22045 Hamburg (DE)
(74) Representative: Seemann & Partner Patentanwälte mbB

(57) **Abstract**

The present invention relates to a control system for controlling an operation performed via a medical instrument, in particular an endoscope, wherein the control system comprises at least one medical instrument configured to be at least partially inserted into a cavity, in particular a cavity in a patient's body, wherein the medical instrument comprises at least one sensor for sensing the cavity, at least one flushing device for flushing the cavity with fluid, and at least one suction device for removing fluid out of the cavity, wherein the control system further comprises a control unit connected to the flushing device and the suction device, and configured to automatically activate the suction device upon activation of the flushing device, and to adjust flushing parameters and suction parameters, such as fluid flow rate, suction force, and flushing duration, based on preset or real-time conditions detected by the sensor. Advanced features such as ranking and dynamically updating flushing and suction parameters may be incorporated, and a machine learning module for adaptive control based on historical and real-time data may be used. The invention ensures precision in fluid management during medical procedures, enhancing operational efficiency and patient safety. The invention also relates to a method for controlling an operation performed via a medical instrument.

## Description

The present invention relates to a control system and a method for controlling an operation performed via a medical instrument, in particular an endoscope.

For patients seeking medical treatment, medical diagnosis is typically one of the first processes performed by healthcare professionals. To obtain such medical diagnosis, various physical examinations may be employed. If the patient suffers from symptoms originating from the inside of the body, specific instruments have to be used, which allow for an examination of internal organs. In such a scenario, endoscopic examination, such as colonoscopy, is a promising tool, as it allows for minimally invasive surgeries.

If a bleeding event occurs during an endoscopic examination or a therapeutic procedure, immediate treatment is required. In case of such an event, it is common to flush the examined cavity with a fluid by using a flushing device comprising a flushing pump. Subsequently, the fluid in combination with body fluids, such as blood, are removed from the cavity by sucking or vacuuming using a suction device comprising a suction pump. Suction of said fluids is needed as they may hinder the ongoing endoscopic examination and harm the patient.

Both flushing device and suction device are commonly operated by manual actuation. To do so, these devices are often provided with a manual switch to control the device, e.g. by actuation of a push button, a foot switch, or a valve. Consequently, the surgeon is required to control each of the devices while at the same time handling the medical instrument used for the endoscopic examination. Furthermore, there is no direct communication between the flushing device and the suction device. Thus, the surgeon must divert his/her focus on both devices and their manual actuation, respectively. Alternatively, a further operator is required to actuate the flushing device and/or the suction device, while the primary surgeon focusses on operating the medical instrument. However, this requires additional qualified medical staff. Even in the case of an actuation mechanism, which is provided directly on the medical instrument, e.g. in the form of a handle configured to activate flushing and suction, the surgeon is required to control the handle while at the same time operating the medical instrument inside the cavity.

In view of the above circumstances, it is an object of the present invention to provide a control system and a method for controlling an operation performed via a medical instrument, which address the above-described problems such that the operation is at least partially automated and the level of complexity is reduced for the surgeon.

According to a first aspect of the present invention, the above object is achieved by a control system for controlling an operation performed via a medical instrument, in particular an endoscope, wherein the control system comprises at least one medical instrument configured to be at least partially inserted into a cavity, in particular a cavity in a patient's body, wherein the medical instrument comprises at least one sensor for sensing the cavity, at least one flushing device for flushing the cavity with fluid, and at least one suction device for removing fluid out of the cavity, wherein the control system further comprises a control unit connected to the flushing device and the suction device, and configured to automatically activate the suction device upon activation of the flushing device, and adjust flushing parameters and suction parameters based on sensor data acquired by the sensor or preset conditions, wherein said parameters include at least one of a flushing duration, a suction force, or a fluid flow rate.

According to a second aspect of the present invention, the above object is further achieved by a control system for controlling an operation performed via a medical instrument, in particular an endoscope, wherein the control system comprises at least one medical instrument configured to be at least partially inserted into a cavity, in particular a cavity in a patient's body, wherein the medical instrument comprises at least one sensor for sensing the cavity, at least one flushing device for flushing the cavity with fluid, and at least one suction device for removing fluid out of the cavity, wherein the control system further comprises a control unit connected to the flushing device and the suction device to automatically activate the suction device upon activation of the flushing device.

According to the first and second aspect of the present invention, the medical instrument may be used for medical treatments, such as endoscopy, to treat patients. In this context, the medical instrument allows for minimal invasive examinations and may thus be employed e.g. for laparoscopic surgery. The medical instrument is preferably an endoscope, but may also be a different type of medical instrument suitable to be at least partially inserted into a cavity. The cavity is in particular a region or area within the human body, e.g. the intestine, stomach, or lung, among others. In general, the medical instrument may be inserted into a cavity through an opening, such as the mouth or anus, to examine internal organs. The medical instrument is destined to be used by a surgeon or any other professional medical staff.

It should be noted that the medical instrument is not limited to medical treatments but may also be utilized in other operations or for other purposes, such as research, e.g. in the context of *in vitro* studies, or training of medical staff using training dummies.

The medical instrument comprises at least one sensor for sensing the cavity. Said sensor may in particular be an image sensor or a camera including a light source for imaging the examined cavity. Image and video data may be transferred in real time to a display unit such that the surgeon may assess the cavity while the medical instrument is inserted into the cavity. Moreover, the medical instrument may be equipped with other sensors, probes or tools, such as fiberoptics, ultrasound devices and micro gripping tools, among others.

Further, the control system comprises at least one flushing device and at least one suction device. At least one of the flushing device and the suction device may comprise a pump. For both the flushing device and the suction device, commercially available devices, in particular pumps, may be employed, e.g. the OFP-2 endoscopic flushing device and the KV-6 endoscopic suction device, both sold by Olympus Europa SE & Co. KG, Germany. Both devices may be individually connected to the medical instrument via tubing, such as to enable flushing of the cavity and suction off the cavity, each via a designated tube. To do so, the medical instrument may comprise at least one first channel connected to the flushing device and at least one second channel connected to the suction device. A fluid, which may be water or any other suitable fluid used in medical applications, is supplied by the flushing device, flows to the medical instrument via the tubing, and is discharged into the cavity to flush or irrigate said cavity. Once the fluid has been discharged into the cavity, the suction device may be activated to remove or extract fluid out of the cavity by suction or vacuuming. Typically, the extracted fluid is a mixture of body fluids, such as blood, and the fluid previously used for flushing.

The control unit can be operatively connected to the medical instrument, the flushing device, and the suction device. In particular, the control unit can be configured to access and process sensor data recorded by the sensor of the medical instrument. The control unit may be a computing device, such as a computer or microcomputer, comprising a processor to process data, and to generate and transmit control commands to devices associated with the control system, in particular to both the flushing and the suction device as well as the medical instrument.

According to an important feature of the first aspect and the second aspect of the present invention, the control unit is configured to automatically activate the suction device upon activation of the flushing device. In other words, the control unit may activate the suction device if it is required to remove fluid from the cavity. Thus, in contrast to conventional systems, the control system takes over control of the suction device such that the surgeon is no longer required to manually actuate said suction device. By doing so, the operation performed via the medical instrument becomes less complex as fewer tasks have to be executed. In particular, the surgeon may focus on operating the medical instrument and the flushing device, without having to pay attention to the activation of the suction device.

In the context is the present invention, the term "upon activation of the flushing device" is to be understood such that the suction device and the flushing device are operated by the control unit in coordination with one another. In particular operation (e.g. activation) of the suction device may be triggered by operation (e.g. activation) of the flushing device. In one embodiment, the control unit may activate the suction device after the flushing device has at least been flushing for a minimum flushing duration. The minimum flushing duration may be established according to reference flushing parameters or may be individually set by the surgeon prior to the activation of the flushing device. The minimum flushing duration may be in the range of 0 s to several seconds, for example more than 2 seconds. In another embodiment, the suction device may be activated simultaneously with the flushing device, while the flushing device is flushing, or after flushing has been executed, i.e., to establish a time delay between flushing and suction.

The term "activated" is to be understood as an initiation of actuation or operation of the respective device, i.e. the suction device starts a suction operation based on preset suction parameters, or may be understood as an increase in operation (e.g. increase in suction power or increase in flushing power/amount). Said suction parameters may comprise at least one of a suction duration, a suction force, a suction capacity, and a power of the suction device. Said flushing parameters, may comprise at least one of a flushing duration, a flushing amount, a flushing strength, a flushing force, and a power of the suction device. A further parameter to be adjusted for both flushing and suction parameters is a ramp up/ramp down of each device. The ramp up/ramp down of each of the flushing device and the suction device may be linear or non-linear depending on the characteristics of the medical application.

Optionally, a flushing duration of the flushing device may be based on manually set flushing parameters or preset flushing parameters input into the flushing device prior to flushing. The flushing parameters may be in particular set with respect to flushing duration and fluid flow output. During an operation performed via the medical instrument, the surgeon may manually activate the flushing device and adjust flushing parameters depending on his/her preferences. Alternatively, the flushing parameters may be preset, such that the surgeon merely needs to manually activate the flushing device, while flushing duration and fluid flow output have already been set prior to the operation.

The control unit is further configured to adjust flushing parameters and suction parameters based on sensor data acquired by the sensor or preset conditions, wherein said parameters include at least one of a flushing duration, a suction force, or a fluid flow rate. According to this feature of the present invention, the flushing device and the suction device may be controlled based on information relating to events occurring in the cavity or based on preset conditions, i.e. conditions relating to the operation performed via the medical instrument or individual settings of the flushing device and/or the suction device. Thus, the control unit is capable of automatically adjusting said flushing parameters and suction parameters, such that the surgeon does not necessarily have to manually set these parameters.

During the operation of the medical instrument, the sensor may detect one or more events occurring within the cavity and may provide sensor data to the control unit, wherein the sensor data may relate to at least one of the detected events. Thus, once an event has been detected by the sensor, the control unit is capable is processing sensor data in real time and output said data such that the surgeon is able to instantly assess any event occurring throughout the operation. E.g., if the sensor is provided in the form of a camera, the control unit may transfer a video signal to a display unit. Alternatively, the medical instrument may directly transfer the sensor data to a display or notification device and simultaneously to the control unit.

The detected event may in particular be at least one of: bleeding, occlusion, fluid contamination, temperature changes, or a combination thereof. Further, such an event may be caused by residuals of stool and an overload of flushing liquids. In the context of the present invention, an "event" is to be understood as any event occurring within the cavity, which requires flushing, as it otherwise might cause harm to the patient and/or hinder the operation performed via the medical instrument. The most common case for such an event is bleeding, which may be caused by certain medical conditions, e.g. gastrointestinal bleeding, or may be caused by the medical instrument itself, either intentionally or unintentionally.

According to a further embodiment of the present invention, the control unit may be configured to automatically and dynamically adjust the suction device based on flushing parameters, wherein the adjustment of the suction device includes at least one of an activation of the suction device, an adjustment of the suction force, and an adjustment of the suction duration. As outlined above, the flushing parameters may either be set and adjusted by the surgeon once flushing is required, or they may be preset. The control unit may be configured to process said flushing parameters and, depending on all or individual flushing parameters, may accordingly adjust the suction device. E.g., if flushing is performed for a duration of 10 s, the control unit may automatically activate the suction device and set a suction duration of more than 10 s to make sure that the entire flushing fluid mixed with body fluids is being removed from the cavity.

Further, the control unit may adjust the suction device while flushing is ongoing, e.g. if flushing parameters are being changed during flushing. The control unit may also take into account that the volume of the fluid to be extracted is increased with respect to the volume of the flushing fluid, as the former may be the combination of flushing fluid and body fluids. Moreover, an adjustment of suction parameters, in particular of the suction force, may be performed by the control unit, e.g. if less fluid than expected is removed from the cavity while suction is ongoing. This may be the case if the viscosity of the fluid increases or relatively small volumes have to be removed. In these cases, the suction force may be increased to remove the remaining fluid from the cavity. The control unit may therefore be provided with sensors to monitor the outflow volume as well as power consumption and resistance of the suction pump, and automatically detect changes related to these parameters. In the same manner, flushing parameters may be adjusted while the flushing is ongoing.

According to another embodiment of the present invention, the control unit may determine and rank the flushing parameters based on at least one of sensor data provided by the sensor, preset or manually input flushing parameters, and real-time events detected during the operation. A ranking of the flushing parameters may further be based on the adjustment of the flushing parameters by the surgeon or on the preset flushing parameters. To determine and generate the ranking of the flushing parameters, the control unit takes into account at least the flushing duration and the fluid flow output. E.g., flushing parameters may be of a higher rank if one or both of the flushing duration and the fluid flow output is higher than an average flushing duration and an average fluid flow output.

The ranking may further be based on reference values obtained from historic data or lookup tables stored in a software database. Individual contributions of flushing duration and fluid flow output are taken into account when establishing the ranking and may be designated accordingly. E.g., if flushing is performed for an extended duration but with a low fluid flow output, the resulting rank will differ from the rank of flushing parameters which comprise average flushing duration and average fluid flow output.

Once a rank has been assigned to the flushing parameters, the control unit may adjust the suction device accordingly, i.e. a higher rank will lead to an extended suction duration and/or increased suction force. This procedure allows for a balanced actuation of the flushing device and the suction device.

Alternatively or in addition to the ranking, the control unit may generate a recommendation on how to adjust the suction device. Thus, based on the recommendation, the surgeon may manually adjust the suction device once the suction device has been automatically activated by the control unit.

Optionally, the control unit constantly and dynamically updates the ranking of the flushing parameters and adjusts the flushing device and the suction device in accordance with the updated ranking. An update of the ranking may become necessary if the surgeon manually adjusts the flushing parameters, e.g. if the fluid flow output is increased, after flushing has already been performed for a certain flushing duration, or if the flushing parameters had previously been automatically adjusted. The suction duration and the suction force may be adjusted according to an updated ranking of the flushing parameters. Thus, the control unit is configured to constantly monitor the activity of both the flushing device and the suction device to detect changes in their respective parameters.

As indicated above, the control unit may be configured to determine flushing parameters based on sensor data provided by the sensor. The sensor data, which comprise information regarding an event occurring within the cavity, is processed by the control unit. The control unit determines an event including characteristics of the event, such as magnitude or intensity, based on the processed sensor data. The control unit may further compare the processed sensor data with historic data or lookup tables stored in a software database to quantify the event.

Once the control unit has detected an event, it may output information regarding said event to the surgeon, e.g. via the display unit. The information may be output in the form of a visual signal, an acoustic signal, or a combination thereof. The output signals may be adapted based on the type of detected event. Further, the visual signal may in particular comprise virtual outlines overlayed over a video or image signal acquired from the sensor. Thereby, the control unit is capable of facilitating the assessment of events occurring within the cavity for the surgeon. Additionally, the control unit may visually display a recommendation for adjusting the flushing parameters and/or suction parameters based on the quantified event.

Moreover, the control unit may be configured to control the flushing device and the suction device by at least one of activating the flushing device, adjusting the fluid flow rate and flushing duration, activating the suction device, and adjusting the suction duration and the suction duration. The activation of the flushing device and the suction device, and their adjustment is in particular performed according to the principles described above. E.g., the control unit may control the flushing device based on the determined flushing parameters. The control unit may further adjust the flushing parameters as well as the suction parameters based on the continuous assessment of sensor data. In case it is detected that a certain event, e.g. bleeding, persists over an extended amount of time, the control unit may extend the flushing duration and/or increase the fluid flow output, and analogously the suction duration and/or the suction force. The control unit may also set flushing parameters prior to flushing.

As a result, the automation of the operation performed via the medical instrument is further increased, as it is no longer required for the surgeon to manually activate and adjust the flushing device and the suction device.

According to a further embodiment of the present invention, the control unit may be configured to, in response to an operation performed via the medical instrument, activate the flushing device, adjust the fluid flow output by the flushing device, adjust the flushing duration of the flushing device, activate the suction device, adjust the suction force of the suction device, and adjust the suction duration of the suction device. Accordingly, a specific type of operation or a specific step within an operation leads to a corresponding control of the flushing device and the suction device. The activation of both devices and the adjustment of flushing parameters and suction parameters is in particular performed according to the principles described above. To determine the type of operation, the control unit may assess operation parameters of the medical instrument. The operation parameters may relate to energy input, operation duration, use of further tools comprised by the medical instrument, such as micro gripping tools, or a combination thereof.

The control unit may further compare the assessed operation parameters with threshold values obtained from historic data or lookup tables stored in a software database. Thereby, the control unit may e.g. activate the flushing device if a certain energy input threshold value has been reached, wherein the energy input into the medical instrument has been determined by the control unit.

According to a further embodiment of the present invention, the control unit may activate or adjust the flushing device and the suction device in response to or based on an operation performed via the medical instrument including at least one of: a laser application, a high-frequency monopolar application, a low-frequency monopolar application, a hot wire application, an electrosurgical intervention, an ultrasonic application, or a combination thereof. The medical instrument may be equipped with designated tools, such as a wire loop, to perform the mentioned applications. E.g., a high-frequency monopolar application of the medical instrument may be intended if a polyp is to be removed from human tissue or a tissue sample is to be collected. In this case, it may be expected that the removal of a poly causes bleeding. Therefore, the control unit may accordingly set flushing parameters and suction parameters once it has assessed the high-frequency monopolar application of the medical instrument. However, since bleeding does not necessarily occur, the control unit may activate the flushing device only if bleeding has actually been detected based on the sensor data.

According to a preferred embodiment of the present invention, the control unit may comprise a machine learning module. Said machine learning module may be configured to record control commands output to the medical instrument, flushing device and suction device, convert recorded commands into training data sets, and use the training data sets to dynamically adjust operation parameters during future operations.

In detail, the machine learning module may in particular comprise a neural network, e.g. a convolutional neural network (CNN). The machine learning module may be controlled by the control unit such as to output control commands for actuating one of the flushing device, the suction device, and the application of the medical instrument based on input values. The input values indicate a determined flushing parameter, suction parameter or operation parameters of the remaining two of the flushing device, the suction device and the application of the medical instrument. The application of the medical instrument is associated with the operation parameters outlined above.

The machine learning module may be trained over an extended period of time by using training data, which are input into the machine learning module. The training data may comprise a plurality of training data sets, wherein each training data set may be obtained by historic data associated with an operation controlled via the medical instrument. In particular, the training data sets comprise parameters, which were controlled and set by surgeons in past operations.

Therefore, each trained data set may reflect control operations, including settings, control commands, and detected parameters of the devices of the control system, for a particular action or a situation during the performed operation. In addition, or alternatively the training data sets may include treatment data and/or auxiliary data which are partially or completely based on theoretical data defined by a surgeon or another person's skill in the art in being suitable for a particular situation during a particular medical treatment. In particular, treatment data may relate to operation parameters, flushing parameters, and suction parameters, while auxiliary data may relate to secondary parameters, such as patient parameters, and time as well as duration of an operation.

In addition, or alternatively the training data sets may comprise weighted training data sets, wherein each training data set comprises treatment data and auxiliary data associated to the treatment data, as well as a weighting factor indicating an assessment of a correlation between the treatment data and the corresponding auxiliary data. The weighting factor may reflect a range of evaluation values ranging from low to high evaluation of the flushing device, the suction device, and the application of the medical instrument, the latter being operated according to the treatment data and the auxiliary data.

The training of the machine learning module is configured such that the devices of the control system perfectly cooperate to master an ongoing operation and perform their individual tasks in an optimal manner from the viewpoint of a surgeon or a person skilled in the art.

After having trained the machine learning module using the procedure described above using a sufficient number of training data sets, the machine learning module is able to predict output values based on unseen input data, i. e. generate treatment data based on unseen auxiliary data, and/or generate auxiliary data based on unseen treatment data. Generated treatment data and/or auxiliary data may then be used by the control unit in the form of control commands to control actuation of the flushing device, the suction device and/or the application of the medical instrument.

According to a further embodiment of the present invention, the control unit may record control commands output to the medical instrument, the flushing device and the suction device, and wherein the control unit converts the recorded control commands into training data sets for training the machine learning module. By doing so, the machine learning module of the control system is constantly trained based on new training data. The control unit may record any control commands, e.g. output values generated by the machine learning module or manually set input parameters by a surgeon.

Further, the flushing device and the suction device may each comprise a flow sensor, wherein the control unit is configured to determine a fluid flow generated by the flushing device and/or the suction device based on fluid flow data provided by the respective flow sensor. Instead or in addition to the determination of a fluid flow or an inflow/outflow volume, the flow sensors may be configured to measure the weight of fluid flushed by the flushing device and extracted by the suction device, respectively. In particular with respect to the suction device, the determination of a fluid weight may be more precise than measuring a fluid volume, as air might be removed from the cavity, which can alter fluid flow measurements.

Optionally, the control unit may be implemented in either the flushing device or the suction device, or may be a standalone control unit. The implementation of the control unit into the flushing device and/or the suction device may be desired if the control system is to be set up in relatively small spaces, e.g. in a small operating room. Alternatively, the control unit may be implemented in a computer, laptop, tablet, smartphone, or other smart devices. In any case, the control unit may be wirelessly connected to the remaining devices, e.g. via Bluetooth or Wi-Fi, given that the remaining devices are capable of wireless communication.

According to a third aspect of the present invention, the above object is further achieved by a method for controlling an operation performed via a medical instrument, in particular an endoscope, wherein the method comprises the steps of inserting at least one medical instrument at least partially into a cavity, wherein the medical instrument comprises at least one sensor for sensing the cavity, flushing, by at least one flushing device, the cavity with fluid, and removing, by at least one suction device, fluid from the cavity, wherein the method further comprises controlling, by a control unit, the flushing device and the suction device to automatically activate the suction device upon activation of the flushing device.

In particular, the method according to the second aspect may be performed using a control system according to the first aspect and second aspect of the present invention. It should be noted that corresponding features, advantages and embodiments described for each of the first, second and third aspect of the present invention may also apply to the other aspects among the first, second and third aspect.

Additional features and advantages of the present invention will become even clearer from the following description of embodiments thereof, when taken together with the accompanying figures, which show in particular:
- Fig. 1: a schematic illustration of a control system including devices associated with the control system according to a first embodiment;
- Fig. 2a, 2b: illustrations of a portion of a medical instrument, wherein the medical instrument is used for flushing and suction, respectively;
- Figs. 3a-3c: diagrams illustrating the relation of various treatment parameters, in particular flushing parameters and suction parameters;
- Fig. 4: an illustration of a portion of a medical instrument, wherein the medical instrument is used for a hot wire application; and
- Fig. 5: a schematic illustration of a control system including devices associated with the control system according to a second embodiment.

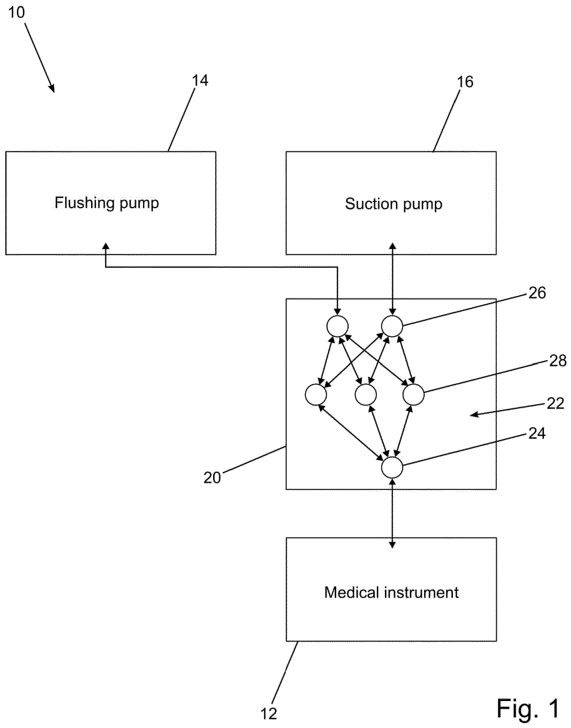

In Figure 1, a schematic illustration of a control system 10 for controlling an operation performed via a medical instrument 12 according to a first embodiment of the present invention is shown. The control system 10 comprises the medical instrument 12, which may in particularly be an endoscope, a flushing device 14 (e.g. a flushing pump) and a suction device 16 (e.g. a suction pump). Further, control system 10 comprises a control unit 20, which is operatively connected to each of the devices of the control system 10, as schematically indicated by arrows. The control unit 20 is configured to send and receive data to/from each of the other devices 12, 14, 16. The control system 10 is adapted for examination and treatment of a patient, e.g. by performing endoscopy.

The medical instrument 12 is configured to be at least partially inserted into a cavity. The cavity may be a cavity in a patient's body, such as the intestine. To do so, the medical instrument 12 is preferably elongated along its main extension direction, has a circular cross section and a diameter in the range of 1 mm to 20 mm. However, the dimensions of the medical instrument 12 may vary depending on the application.

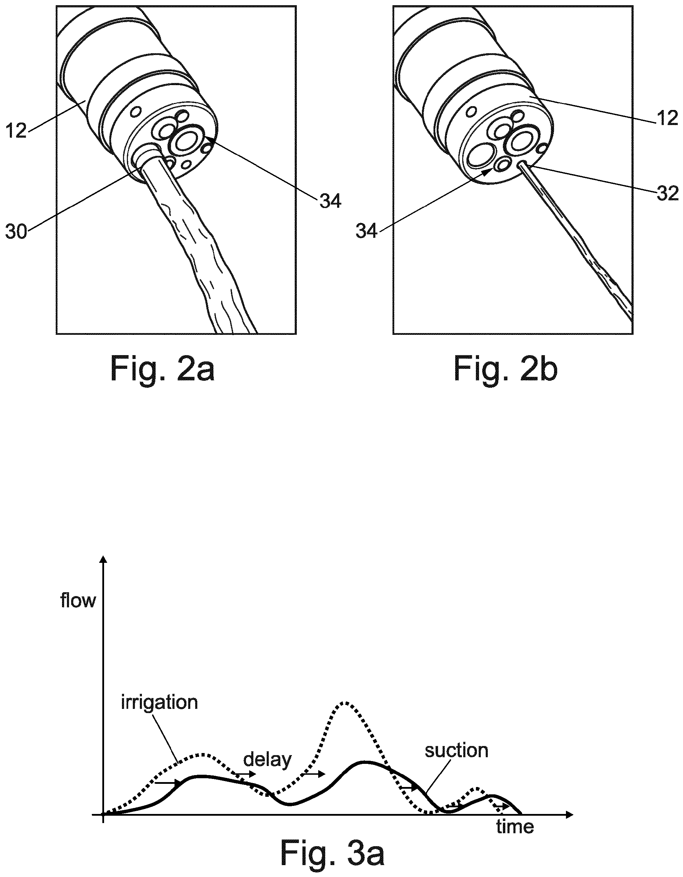

As shown in Figures 2a und 2b, the medical instrument 12 comprises a plurality of channels 30, 32, 34, which extend throughout the elongated body of the medical instrument 12. The channels 30, 32, 34 are used to obtain specific functionalities of the medical instrument 12. A first channel 30, which is also referred to as instrument channel, may be used for suction. In detail, the first channel 30 shown in Figure 2a is connected to a suction device 16 via tubing (not shown). By actuating the suction device 16, fluid present in an examined cavity may be extracted into the first channel 30 and further into a reservoir of the suction device 16 via the tubing.

In Figure 2b, a second channel 32, which is also referred to as auxiliary channel, is indicated. The second channel 32 is used for flushing or irrigation of the cavity, e.g. in case bleeding is detected within the cavity. Analogously to the first channel 30, the second channel 32 is connected to a device via tubing, in this case to a flushing device 14 configured to provide a fluid for flushing the cavity.

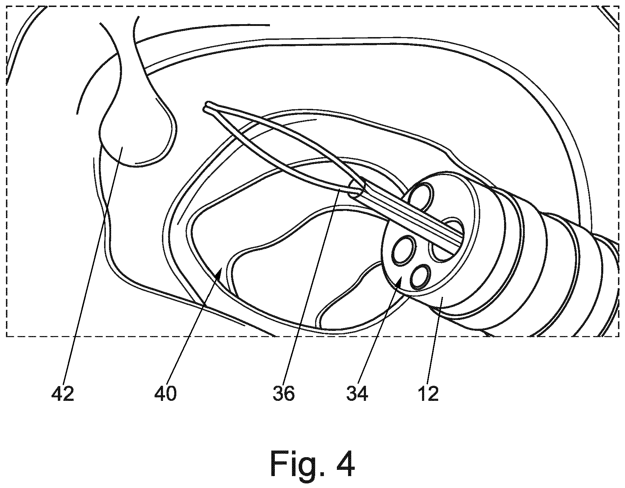

The medical instrument 12 further comprises a plurality of third channels 34, which may have varying functionalities depending on the application. One of the channels 34 is designated for a sensor, in particular an imaging sensor or a camera. Another channel 34 comprises a light source for illuminating the examined cavity. Finally, a further channel 34 may comprise micro tools, such as micro gripping tools or a hot wire 36 for removing polyps 42 from the inside of the cavity 40, as shown in Figure 4. Optionally, the instrument channel 30 may be used to provide said micro tools.

Moreover, the medical instrument 12 is connected to an energy source (not shown). The energy source is configured to supply energy to the medical instrument 12 required for standard operation of the medical instrument 12. Further, the energy source in combination with at least one of the channels 30, 32, 34 of the medical instrument 12 may enable various operational applications, such as laser applications or hot wire applications. More specifically, the energy source supplies specific energy levels required for a given application to the medical instrument 12 and a matching tool thereof, e.g. the hot wire 36 shown in Figure 4.

Coming back to Figure 1, the flushing device 14 and the suction device 16 comprise user input means, such as a foot switch, handles or push buttons for manually activating the respective device 14, 16 and means for adjusting specific device parameters, such as an actuation duration, fluid flow, force, and ramp up/ramp down, among others. Thus, a surgeon performing an operation via the medical instrument 12 is able to actuate the flushing device 14 and the suction device 16.

In addition, the control unit 20 is configured to control the actuation of both the flushing device 14 and the suction device 16, such that the operation is at least partially automated, and the surgeon is no longer required to manually actuate said devices 14, 16. In particular, the control unit 20 automatically activates the suction device 16 upon activation of the flushing device 14. The control unit 20 may determine flushing parameters and suction parameters based on data provided by the sensor and/or based on energy levels supplied by the energy source.

According to the first embodiment of the present invention, it is possible to reduce the time required to perform the operation, as the surgeon may focus primarily on handling the medical instrument 12. As the control unit 20 is configured to control both the flushing device 14 and the suction device 16, events occurring throughout the operation, such as bleeding, may be detected faster and in a more reliable manner. Further, due to the automation, the usage of flushing fluid may be optimized. I.e., the volume of flushing fluid used during flushing may be automatically adjusted by the control unit 20 to prevent using excessive amounts of flushing fluid.

The control unit 20 is configured to set and adjust flushing parameters of the flushing device 14, suction parameters of the suction device 16, and operation parameters of the medical instrument 12, based on sensor data acquired by the sensor or preset conditions of the devices, wherein said parameters include at least one of a flushing duration, a suction force, or a fluid flow rate. As all devices of the control system 10 are in communication via the control unit 20, data exchange between the devices 12, 14, 16 is possible. Said data in particular refers to flushing parameters, suction parameters, operation parameters, and sensor data, but also to historic data associated with an operation performed via the medical instrument 12. The control unit 20 may employ an algorithm, which correlates the individual parameters with each other. E.g., a suction duration and a suction force may be adjusted based on flushing parameters, operation parameters and/or sensor data.

A display unit (not shown) is optionally also comprised by the control system 10 according to the first embodiment. The display unit outputs image and video data acquired by the sensor of the medical instrument 12, such that a surgeon is able to assess the examined cavity in real time.

As shown in Figure 1, the control unit 20 may comprise a machine learning module 22 in the form of a neural network and at least one input layer 24, at least one output layer 26 and one or more hidden layers 28 between the input layer 24 and the output layer 26 as conventionally known. In the first embodiment, the input layer 24 is connected to and receives input from the medical instrument 12 including the energy source, while the output layer 26 is connected to and outputs data to each of the flushing device 14 and the suction device 16.

Training data may be input into the machine learning module 22, wherein the data is obtained based on flushing parameters, suction parameters and/or parameters with respect to an application of the medical device 12. The training data stems from historic data associated with past operations or control commands issued by the control unit 20.

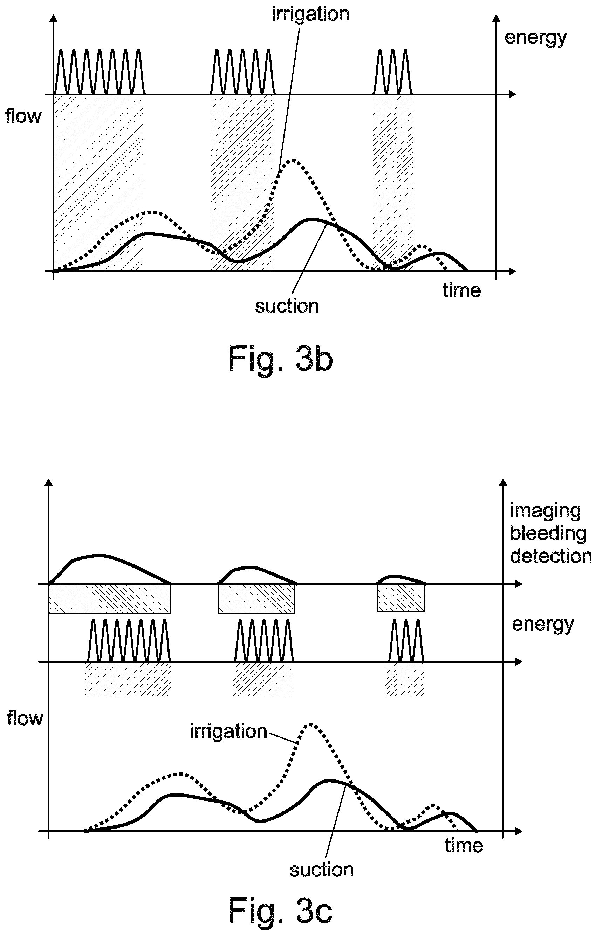

Reference is now made to Figures 3a, 3b, and 3c, which show diagrams illustrating a relation of various treatment parameters, in particular flushing parameters, suction parameters, and operation parameters.

The control unit 20 may activate, control and dynamically adjust the suction device 16 based on present flushing parameters (irrigation), e.g. to treat bleeding within the cavity. Thus, the flushing device 14 and the suction device 16 are in a master-slave relationship, as shown in Figure 3a. While the flushing device 14 and the suction device 16 may be either simultaneously activated or with a delay, the suction force of the suction device 16 is slowly ramped up relative to the fluid flow output of the flushing device 16. In particular, the ramp up of the suction force is delayed relative to the ramp up of the fluid flow output to prevent fluid from being immediately removed before the cavity has been properly flushed. Further, excessive suction may cause damage to the tissue of the patient and is therefore to be avoided. As can be seen from the diagram of Figure 3a, the suction force is constantly adjusted relative to the fluid flow output until flushing/irrigation has been stopped. To determine suction parameters and flushing parameters according to the example shown in Figure 3a, both the suction device 16 and the flushing device 14 comprise flow sensors, which measure inflow and outflow of the corresponding device 14, 16 and communicate the resulting flow information to the control unit 20.

Figure 3b highlights the adjustment of flushing parameters (irrigation) and suction parameters relative to an energy-based application of the medical instrument 12, e.g. a hot wire application for removing a polyp 42. The flushing device 14 and the suction device 16 may be automatically activated by the control unit 20 based on the energy level supplied by the energy source. The activation of said devices 14, 16 and the adjustment of their respective flushing parameters and suction parameters may depend on operation parameters of the energy-based application, e.g. duration, power, frequency, and pulse type, among others. Depending on the application and in particular on the operation parameters of the application, control of the flushing device 14 and the suction device 16 may vary. E.g. flushing device 14 and suction device 16 may be simultaneously activated upon initiation of the application or once a poly 42 has been cut. With respect to the relation of flushing/irrigation to suction, reference is made to Figure 3a.

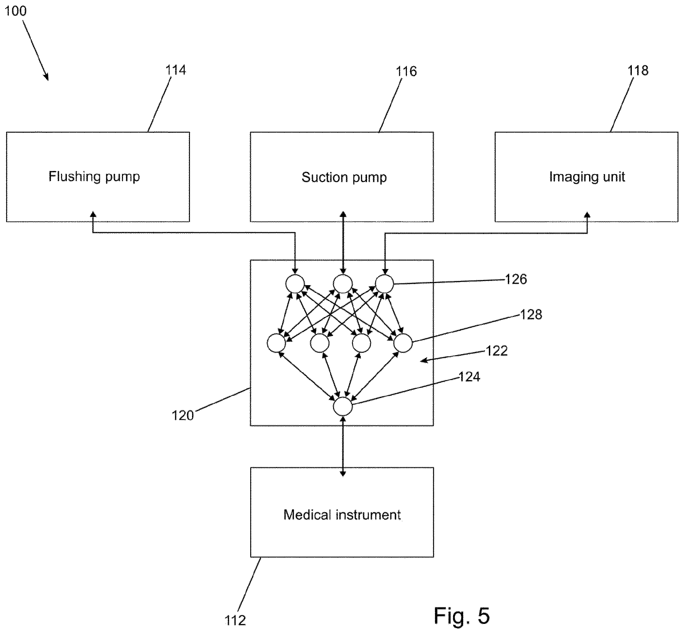

Finally, Figure 3c shows the adjustment of flushing parameters (irrigation) and suction parameters relative to an energy-based application of the medical instrument 112 in combination with an imaging unit 118, according to a second embodiment of the present invention, illustrated in Figure 5.

In case of an energy-based application of the medical instrument 112 as described with respect to Figure 3b, the imaging unit 118 may be employed to determine and quantify bleeding within the examined cavity 40. As mentioned above, the imaging unit 118 outputs visual signals, preferably in the form of video signals, based on sensor data provided by the sensor of the medical instrument 112.

If bleeding or any other event, which requires flushing of the cavity 40, is detected and quantified, the flushing device 114 and the suction device 116 are activated and their respective parameters are adjusted based on the principles outlined above. Further, the operation parameters relating to the energy-based application of the medical instrument 112 may be adjusted. Said adjustment may refer to an ongoing operation or may be directed towards future operations, e.g. by generating training data. Thus, future energy-based applications may be optimized. In particular, the operation parameters may be optimized with respect to coagulation and to reduce energy consumption to a necessary amount, such that tissue damage, carbonization, or other detrimental effects may be avoided.

In Figure 5, the control system 100 according to the second embodiment of the present invention is shown. Reference numerals of devices, which are present in both the first and the second embodiment, were incremented by 100. For devices 112, 114, 116, reference is made to the first embodiment described above.

With respect to the machine learning module 122 of the control unit 120, an additional output layer 126 is present, which is associated with imaging unit 118. Thus, the connection between input layer 124, hidden layers 128 and output layers 126 is extended based on the new output layer 126. The machine learning module 122 of the second embodiment is trained based on and configured to receive imaging signals from the imaging unit 118 in addition to sensor data from the medical instrument 112. As a result, the control unit 120 is able to control all of the devices comprised by the control system 100, i.e. the medical instrument 112, the flushing device 114, the suction device 116, and the imaging unit 118, by issuing control commands based on output by the machine learning module 122.

## Claims

1. Control system for controlling an operation performed via a medical instrument, in particular an endoscope, wherein the control system comprises:
at least one medical instrument configured to be at least partially inserted into a cavity, in particular a cavity in a patient's body, wherein the medical instrument comprises at least one sensor for sensing the cavity;
at least one flushing device for flushing the cavity with fluid; and
at least one suction device for removing fluid out of the cavity,
**characterized in that**
the control system further comprises a control unit connected to the flushing device and the suction device, and configured to:
automatically activate the suction device upon activation of the flushing device; and
adjust flushing parameters and suction parameters based on sensor data acquired by the sensor or preset conditions, wherein said parameters include at least one of a flushing duration, a suction force, or a fluid flow rate.

2. Control system according to claim 1, wherein the control unit is configured to dynamically adjust the suction device based on flushing parameters, wherein the adjustment of the suction device includes at least one of:
- activation of the suction device;
- adjustment of the suction force; and
- adjustment of the suction duration.

3. Control system according to claim 1 or 2, wherein the sensor detects events occurring within the cavity and provides sensor data to the control unit, and wherein the events include at least one of: bleeding, occlusion, fluid contamination, temperature changes, or a combination thereof.

4. Control system according to any of the preceding claims, wherein the control unit determines and ranks flushing parameters based on at least one of:
- sensor data provided by the sensor;
- preset or manually input flushing parameters; and
- real-time events detected during the operation.

5. Control system according to claim 4, wherein the ranking of the flushing parameters is dynamically updated, and the control unit adjusts the flushing device and suction device based on the updated ranking.

6. Control system according to any of the preceding claims, wherein the control unit activates or adjusts the flushing device and the suction device in response to an operation performed via the medical instrument, including at least one of:
- a laser application;
- a high-frequency monopolar application;
- a low-frequency monopolar application;
- a hot wire application; and
- an electrosurgical intervention.

7. Control system according to any of the preceding claims, wherein the control unit comprises a machine learning module configured to:
- record control commands output to the medical instrument, flushing device, and suction device;
- convert recorded commands into training data sets; and
- use the training data sets to dynamically adjust operation parameters during future operations.

8. Control system according to any of the preceding claims, wherein the control unit is configured to control the flushing device and the suction device by at least one of:
- activating the flushing device;
- adjusting the fluid flow rate and flushing duration;
- activating the suction device; and
- adjusting the suction force and duration.

9. Control system according to any of the preceding claims, wherein the flushing device and suction device each comprise a flow sensor, and the control unit determines fluid flow based on data from the flow sensors.

10. Control system according to any of the preceding claims, wherein the control unit is implemented in either the flushing device or the suction device, or is a standalone control unit.

11. Method for controlling an operation performed via a medical instrument, in particular an endoscope, wherein the method comprises the following steps:
- inserting at least one medical instrument at least partially into a cavity, wherein the medical instrument comprises at least one sensor for sensing the cavity;
- flushing the cavity with fluid, by at least one flushing device; and
- removing fluid from the cavity, by at least one suction device;
**characterized in that**
the method further comprises:
a control unit, which automatically activates the suction device upon activation of the flushing device, and which adjusts flushing parameters, suction parameters, and operation parameters based on sensor data or preset conditions.

12. Method according to claim 11 using a control system according to any of claims 1 to 11.
